# EUROPEAN PATENT APPLICATION

(11) **EP 4 851 143 A1**
(43) Date of publication of application: **09.12.2026**
(21) Application number: 25747849.5
(22) Date of filing: 24.01.2025
(51) Int. Cl.: C07D 307/62, A61K 8/67

(54) **SOLID FORM OF ASCORBIC ACID CARBON CHAIN DIBASIC ACID ESTER AND USE THEREOF**

(30) Priority: 30.01.2024 US 202463626547 P
(71) Applicant: Corum Inc., Taipei City (TW)
(72) Inventor: HSU, Nai-Hsuan, Taipei City 114 Taiwan (CN); WU, Pei-Hsuan, Taipei City 114 Taiwan (CN); WEN, Liang-Han, Taipei City 114 Taiwan (CN)
(74) Representative: Viering, Jentschura & Partner mbB Patent- und Rechtsanwälte
(86) International application number: PCT/CN2025/074771
(87) International publication number: WO 2025/162281

(57) **Abstract**

A solid form of dibasic acid ester of ascorbic acid, and its application are disclosed. Specifically, the solid form of dibasic acid ester of ascorbic acid comprises at least 90.wt% of bis-(L-ascorbic acid-6,6-)nonanedioate relative to total weight of the solid form, and have a X-ray powder diffraction (XRPD) pattern as shown in Figure 1.

## Description

### TECHNICAL FIELD

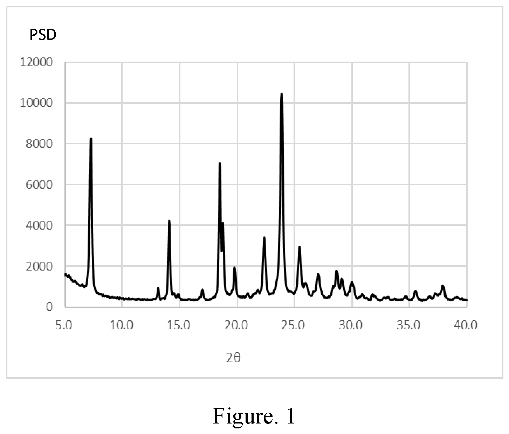

A solid form of dibasic acid ester of ascorbic acid and its applications are disclosed. Specifically, the solid form of dibasic acid ester of ascorbic acid has a XPRD pattern as shown in figure. 1.

### BACKGROUND

In the prior art, ascorbic acid fatty acid ester derivatives are mostly used as additives in the food industry. These ascorbic acid fatty acid esters are almost insoluble in water or polyols and their powder form is unstable. These factors constitute technical barriers to their application in other industries, such as skincare industry.

To overcome the technical hurdles, developing an innovative ascorbic acid derivative that can be applied to the skincare industry is a topic that urgently needs to be researched and broken through in this technical field.

### SUMMARY OF THE INVENTION

Herein, a dibasic acid ester of ascorbic acid and a diascorbyl alkyl ester described in the following invention descriptions and embodiments are synonyms with equivalent meanings and represent the same chemical structure.

Herein, a solid form of dibasic acid ester of ascorbic acid of this invention comprises at least 90 wt.% of bis-(L-ascorbic acid-6,6-)nonanedioate relative to total weight of the solid form.

Herein, the appearance properties of the solid form described in the following descriptions and embodiments include, but are not limited to lumps, powders, crystals, or their mixtures thereof

Based on the background, the objective of this invention is to provide a thermally stable solid form of the dibasic acid ester of ascorbic acid and its applications in achieving the skin care effects of protecting dermal fibroblasts or keratinocytes from UV damage and promoting collagen production by dermal fibroblasts or keratinocytes through the thermally stable solid form of the dibasic acid ester of ascorbic acid.

In one aspect, the invention is to provide a solid form of the dibasic acid ester of ascorbic acid, wherein the X-ray powder diffraction (XRPD) pattern of the solid form of the dibasic acid ester of ascorbic acid that comprises following characteristic peaks expressed in 2θ degree: 7.2 ±0.2°, 14.1 ±0.2°, 18.5 ±0.2°, 18.7 ±0.2°, 19.8 ±0.2°, 22.4 ±0.2°, 23.9 ±0.2° and 25.4 ±0.2°.

In one aspect, the solid form of the dibasic acid ester of ascorbic acid has a FTIR spectrum that comprises characteristic peaks at 3416±2 ^{cm-1},3172±2 ^{cm-1},2928±2 ^{cm-1}, 2857±2 ^{cm-1}, 1749±2 ^{cm-1}, 1737±2 ^{cm-1}, 1681±2 ^{cm-1}, 1347±2 ^{cm-1}, 1305±2 ^{cm-1}, 1146±2 ^{cm-1} and 761±2 ^{cm-1}.

In one aspect, the solid form of the dibasic acid ester of ascorbic acid has a DSC curve with one endotherm peak between 150 and 160°C.

In another aspect, the invention is to provide a skincare composition that comprises 0.0001~20wt.% of the solid form of the dibasic acid ester of ascorbic acid as recited in the mentioned-above and 80~99.9999wt.% of a base relative to total weight of the skincare composition, and water activity value (aw) of the base is equal to or less than 0.7.

In another aspect, the base is a form selected from the group consisting of water in oil, water in silicon, anhydrous gel and gel.

In another aspect, the base comprises following ingredients: plant oils, mineral oils, polyols, silanes or their combinations.

In still another aspect, the invention is to provide a method for managing skin health. The method comprises topically applying a formulation to a surface of a skin in need, wherein the formulation comprises 0.0001~20wt.% of the solid form of the dibasic acid ester of ascorbic acid as recited in the mentioned-above relative to total weight of the formulation, and water activity value (aw) of the formulation base is equal to or less than 0.7.

In still another aspect, the formulation is selected from the group consisting of cream, lotion, gel, powder and paste.

In still another aspect, the formulation is used for preventing fibroblasts or keratinocytes from UV damage.

In still another aspect, the solid form of dibasic acid ester of ascorbic acid is used for stimulating fibroblasts or keratinocytes to produce collagens.

Accordingly, the technical means and effects of the present invention include, but are not limited to: providing a thermally stable solid form of the dibasic acid ester of ascorbic acid; achieving the technical effect of stabilizing the solid form of the dibasic acid ester of ascorbic acid by means of controlling the water activity value of the skincare composition; and achieving the effect of protecting fibroblasts or keratinocytes from ultraviolet damage and promoting production of collagen by topically applying a composition that comprises the thermally stable solid form of the dibasic acid ester of ascorbic acid in a surface of a skin in need.

### BRIEF DESCRIPTION OF THE DRAWINGS

Figure 1 is the XRPD pattern of the solid form of the of the dibasic acid ester of ascorbic acid.

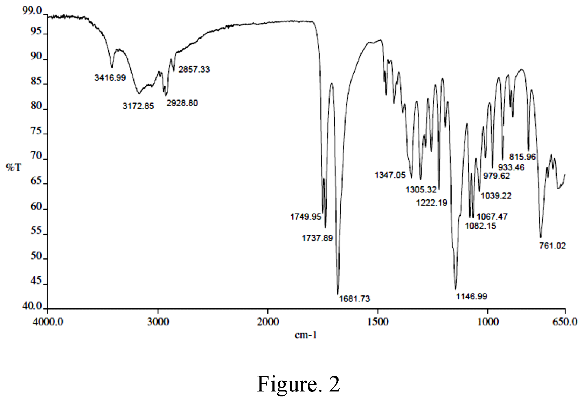

Figure 2 is the FTIR spectrum of the thermally stable solid form of the of the dibasic acid ester of ascorbic acid.

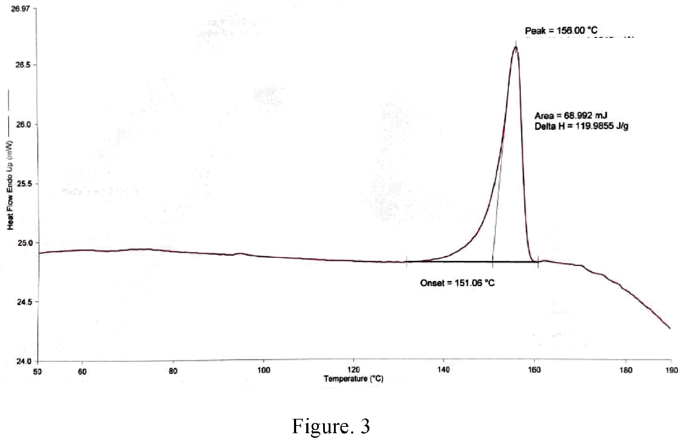

Figure 3 is the DSC curve of the solid form of the of the dibasic acid ester of ascorbic acid.
Figure 4 is the XRPD pattern of the solid in example 3.
Figure 5 is the XRPD spectrum of the solid form of the of dibasic acid ester of ascorbic acid after being stored in an oven at 45°C for 1 month.
Figure 6 is the XRPD spectrum of the solid in example 3 after being stored in an oven at 45°C for 1 month.
Figure 7 is a bar graph showing the effect of the solid form of the dibasic acid ester of ascorbic acid on the recovery of cell viability after UV irradiation.
Figure 8 is a bar graph showing the effect of the solid form of the dibasic acid ester of ascorbic acid on the reduction of intracellular ROS content in cells after UV irradiation.
Figure 9 is a bar graph showing the effect of the solid form of the dibasic acid ester of ascorbic acid on the reduction of intracellular IL-6 content in cells after UV irradiation.
Figure 10 is a bar graph showing the effect of the solid form of the dibasic acid ester of ascorbic acid on increasing collagen expression in dermal fibroblasts.

### EMBODIMENTS

In the first embodiment, a solid form of the dibasic acid ester of ascorbic acid is disclosed. Specifically, the solid form of the dibasic acid ester of ascorbic acid comprises at least 90 wt.% of bis-(L-ascorbic acid-6,6-)nonanedioate relative to total weight of the solid form.

In one embodiment, the solid form of the dibasic acid ester of ascorbic acid comprises at least 95 wt.% of bis-(L-ascorbic acid-6,6-)nonanedioate relative to total weight of the solid form.

In one best embodiment, the solid form of the dibasic acid ester of ascorbic acid comprises at least 99 wt.% of bis-(L-ascorbic acid-6,6-)nonanedioate and less than 1 wt.% of ascorbic acid relative to total weight of the solid form.

In another embodiment, the solid form of the dibasic acid ester of ascorbic acid comprises less than or equal to 5 wt.% of bis-(L-ascorbic acid-5,6-) nonanedioate, bis-(L-ascorbic acid-5,5-) nonanedioate, or their combinations relative to total weight of the solid form.

In another embodiment, the solid form of the dibasic acid ester of ascorbic acid comprises less than or equal to 5 wt.% of L-ascorbic acid-6-nonanedioate, L-ascorbic acid-5-nonanedioate, or their combinations relative to total weight of the solid form.

In one preferred embodiment, the solid form of the dibasic acid ester of ascorbic acid comprises at least 90.wt% of bis-(L-ascorbic acid-6,6-)nonanedioate relative to total weight of the solid form, and has a X-ray powder diffraction (XRPD) pattern that comprises characteristic peaks expressed in 2-theta degree at 7.2±0.2°, 14.1+0.2°, 18.5±0.2°, 18.7±0.2°, 19.8±0.2°, 22.4±0.2°, 23.9±0.2° and 25.4±0.2°.

In one preferred the embodiment, the solid form of the dibasic acid ester of ascorbic acid comprises at least 90.wt% of bis-(L-ascorbic acid-6,6-)nonanedioate relative to total weight of the solid form and has a FTIR spectrum that comprises characteristic peaks at 3416±2 ^{cm-1},3172±2 ^{cm-1},2928±2 ^{cm-1}, 2857±2 ^{cm-1}, 1749±2 ^{cm-1}, 1737±2 ^{cm-1}, 1681±2 ^{cm-1}, 1347±2 ^{cm-1}, 1305±2 ^{cm-1}, 1146±2 ^{cm-1} and 761±2 ^{m-1}.

In one preferred embodiment, the solid form of the dibasic acid ester of ascorbic acid comprises at least 90.wt% of bis-(L-ascorbic acid-6,6-)nonanedioate relative to total weight of the solid form and has a DSC curve with one endotherm peak between 150 and 160°C.

In the second embodiment, a skincare composition was disclosed. The skincare composition comprises 0.0001~20wt.% of the solid form of the dibasic acid ester of ascorbic acid as mentioned above and 80~99.9999wt.% of a base relative to total weight of the skincare composition, and water activity value (aw) of the base is equal to or less than 0.7. Preferably, water activity value (aw) of the base is less than 0.6.

In one embodiment, the base with a water activity value less than 0.6 comprises, but is not limited to: gelatin, fatty acids (such as trade name MBK ^{™}), mixtures containing polyethylene glycol and mineral oil (such as trade name Plasticized ^{™}), mixtures containing silicone oil and vegetable oil (such as trade name PracaSil^{®}-Plus), mixtures containing phospholipids (such as trade name Anhydrous Lipoderm^{®}), and hydrogel-free materials (such as trade name PermE8 ^{™}) or WO6 ^{™} Anhydrous Topical Gel, a mixture containing emulsified wax and fatty acid esters (such as the trade name VersaBase Cream), or a similar composition as mentioned above.

In one embodiment, the skincare composition comprises 0.1 to 20 wt.% of the solid form of dibasic acid ester of ascorbic acid as mentioned above relative to total weight of the skincare composition. Preferably, the skincare composition comprises 1 to 10 wt.% of the solid form of the dibasic acid ester of ascorbic acid as mentioned above relative to total weight of the skincare composition.

In one embodiment, the base is a form selected from the group consisting of water in oil, water in silicon, anhydrous gel and gel.

In one preferred embodiment, water activity value of the water-in-oil (W/O) base is between 0.5 and 0.65 the water activity value of the anhydrous hydrogel or gel is between 0.4 and 0.7.

In one embodiment, the plant oil comprises jojoba oil, black cumin oil, grape seed oil, sunflower seed oil, olive oil, avocado oil, argan oil, macadamia oil, sweet almond oil, apricot kernel oil, sesame oil, bakas fruit oil, or a combination thereof.

In one embodiment, the polyol comprises glycerol, butanediol, 1,3-propanediol, 1,2-propanediol, 1,2- pentanediol, 1,2-hexanediol, polyethylene glycol, poly(1,3 - propanediol), cyclodextrin and its derivatives, cellulose and its derivatives, or their combination thereof.

In one embodiment, the silane comprises polydimethylsiloxane, cyclopentadimethylsiloxane, or their combination thereof.

In one embodiment, the base further comprises pH buffers (such as sodium citrate buffer), antioxidants (such as vitamin E or sodium bisulfite), thiols (such as cysteine or cystamine), chelating agents (such as ethylenediaminetetramethylenephosphonic acid (EDTMP) or its salts), water or their combination thereof.

In a third embodiment, a method for managing skin health was provided. The method comprises a step of topically applying a formulation to a surface of a skin in need, wherein the formulation comprises 0.0001~20wt.% of the solid form of the dibasic acid ester of ascorbic acid relative to total weight of the formulation, and water activity (aw) of the formulation base is equal to or less than 0.7. Preferably, the formulation comprises 0.1~10wt.% of the solid form of the dibasic acid ester of ascorbic acid relative to total weight of the formulation.

In one embodiment, the formulation is selected from the group consisting of cream, lotion, gel, powder, paste, mask liquid, and serum.

In one embodiment, the formulation is used for preventing fibroblasts or keratinocytes from UV damage.

In another embodiment, the solid form of the dibasic acid ester of ascorbic acid is used for stimulating fibroblasts or keratinocytes to produce collagens.

In one embodiment, the solid form of the dibasic acid ester of ascorbic acid has the function of protecting skin fibroblasts or keratinocytes from UV damage.

In one specific embodiment, the solid form of the dibasic acid ester of ascorbic acid has the effect of preventing and repairing UV-induced cell damage with an effective concentration is greater than 125 ppm.

In one specific embodiment, the solid form of the dibasic acid ester of ascorbic acid has a significant effect on reducing intracellular reactive oxygen species with an effective concentration is greater than 125 ppm.

In one specific embodiment, the solid form of the dibasic acid ester of ascorbic acid has a significant effect in reducing intracellular inflammatory mediators with an effective concentration greater than 125 ppm.

In one embodiment, the solid form of the dibasic acid ester of ascorbic acid is used for promoting collagen production by dermal fibroblasts or keratinocytes. In a specific embodiment, the solid form of the dibasic acid ester of ascorbic acid is to increase collagen expression in fibroblasts with an effective concentration is greater than 1 ppm.

### Example 1 : Preparation of the dibasic acid ester of ascorbic acid

L-ascorbic acid (3 equivalents), azelaic acid (1 equivalent), and lipase (20 wt.% based on total weight of reactants) were added to an aprotic reaction solvent. The reaction was carried out under nitrogen at 60~80 °C. After the reaction was complete, the lipase was removed by filtration, excess ascorbic acid was removed by washing with water, and the aprotic reaction solvent was removed by concentration to obtain a crude. Use column chromatography to purify the crude for obtaining a solid form of the dibasic acid ester of ascorbic acid. HPLC analysis showed the solid form comprises at least 90 wt.% of bis-(L-ascorbic acid-6,6-) nonanedioate relative to total weight of the solid form, with a residual ascorbic acid content of 0.62wt.%. Mass spectrometry (MS-ESI): 503.20; ¹H NMR analysis data are shown in Table 1 and its molecular formula is C₂₁H₂₈O₁₄.

**Table 1**

| No | Chemical shift (ppm) | Integral | H number |
|---|---|---|---|
| 1 | 1.2627 | 3.2023 | 6 |
| 2 | 1.5120~1.5349 | 2.0486 | 4 |
| 3 | 2.3030~2.3277 | 2.0033 | 4 |
| 4 | 3.3355 | 1.6204 | d-DMSO |
| 5 | 3.9454~3.9703 | 1.0194 | 2 |
| 6 | 4.0296~4.0917 | 2,0362 | 4 |
| 7 | 4.6661~4.6691 | 1.000 | 2 |
| 8 | 5.2973 | 0.8223 | 2 |
| 9 | 8.4031 | 0.7461 | 2 |
| 10 | 11.0719 | 0.6976 | 2 |

### Example 2 : Recrystallization from ester solvent

The solid form comprises at least 90 wt.% of bis-(L-ascorbic acid-6,6-) nonanedioate prepared by example 1 was recrystallized with at least 4 times weight of a hot non-ester solvent, such as ethyl acetate, relative to weight of the solid form. After cooling and filtration, a wet cake was obtained and then dried under vacuum to prepare a new solid form of the dibasic acid ester of ascorbic acid. HPLC analysis showed that its composition conformed to the range described in example 1. The structure of the new solid form was analyzed by using an X-ray Diffractometer, and its XRPD pattern is shown in Figure 1.

### Example 3 : Recrystallization from non-ester solvent

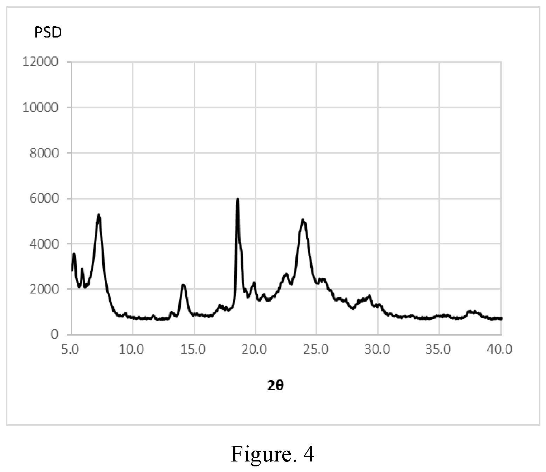

The solid form comprises at least 90 wt.% of bis-(L-ascorbic acid-6,6-) nonanedioate prepared by example 1 was recrystallized with at least 4 times weight of a hot non-ester solvent, such as acetone, relative to weight of the solid form. After cooling and filtration, a wet cake was obtained and then dried under vacuum to prepare another new solid form of the dibasic acid ester of ascorbic acid. HPLC analysis showed that its composition conformed to the range described in example 1. The structure of another new solid form was analyzed by using an X-ray Diffractometer, and its XRPD pattern is shown in Figure 4.

### Example 4: Thermal Stability of the solid form

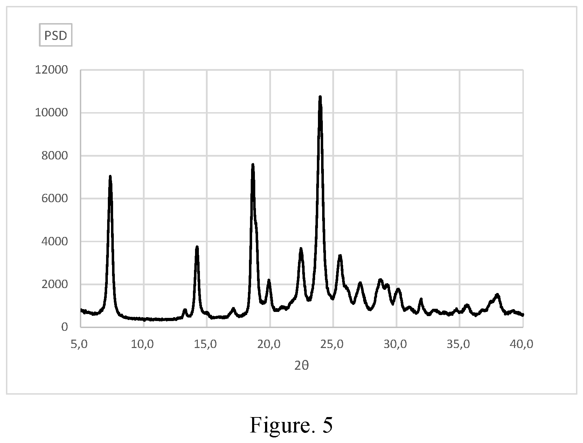

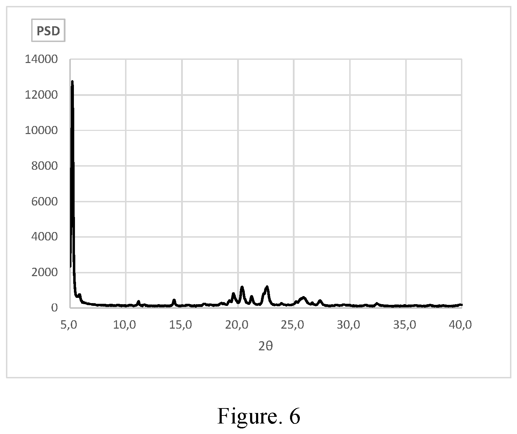

The solid form of the dibasic acid ester of ascorbic acid prepared by recrystallization in examples 2 and 3 were placed in an oven at 45°C for one month, and then analyzed their purity and solid form. HPLC analysis showed that their compositions were still within the range described in example 1. X-ray powder diffraction (XRPD) was used to analyze their solid forms, and the XRPD patterns are shown in Figures 5 and 6, respectively. Comparing figure 1 and 5, the results show that the solid form of the dibasic acid ester of ascorbic acid obtained by recrystallization using ester solvents has high thermal stability, and its structure does not change due to heat, thus avoiding deterioration. In contrast, figure 4 and 6 show significant differences, indicating that the solid form of the dibasic acid ester of ascorbic acid obtained by recrystallization using ether or ketone solvents that do not undergo transesterification with ascorbic acid esters has a poorer thermal stability. Accordingly, the solid form of the dibasic acid ester of ascorbic acid obtained by recrystallization using ester solvents has an excellent structural thermal stability.

### Example 5: Efficacy Experiment 1 (Cell Viability)

Cells were seeded into 48-well plates and cultured at 37°C with 5% CO2 for 24 hours. The cell line used was epidermal cells (HaCaT). (AddexBio, Cat#T0020001); the cell culture medium was DMEM medium containing 10% fetal bovine serum. The cell culture medium was replaced with a buffer solution [ Sterile D-PBS (Dulbecco's phosphate buffered saline)], and the cells were irradiated with UV. The experimental group used a buffer solution containing the solid form of dibasic acid ester of ascorbic acid of this invention, while the basal and control groups used buffer solutions without the solid form of dibasic acid ester of ascorbic acid of this invention. Only the experimental group and the control group received UV irradiation; the basal group was under normal culture conditions, with cells completely covered with aluminum foil to avoid UV irradiation. The post-UV irradiation treatment steps were as follows: the buffer solution was replaced with cell culture medium. The experimental group used cell culture medium containing the solid form of dibasic acid ester of ascorbic acid of this invention, while the basal and control groups used cell culture medium without the solid form of dibasic acid ester of ascorbic acid of this invention. Both groups were cultured at 37°C and 5% CO2 for 24 hours. A cell viability assay was then performed. Cell viability in each experimental group was detected using an AlamarBlue assay, and cell survival rate was calculated using Equation 1. The basal group was cultured under normal conditions; therefore, the cell survival rate of the basal group was defined as 100%. Comparisons between groups were performed using an unpaired Student's t-test.$\begin{matrix}Cell Viability \left(%\right) \\ = \frac{Measured value of basal , control or experimental group}{Measured value of control group} \times 100 %\end{matrix}$ Equation 1

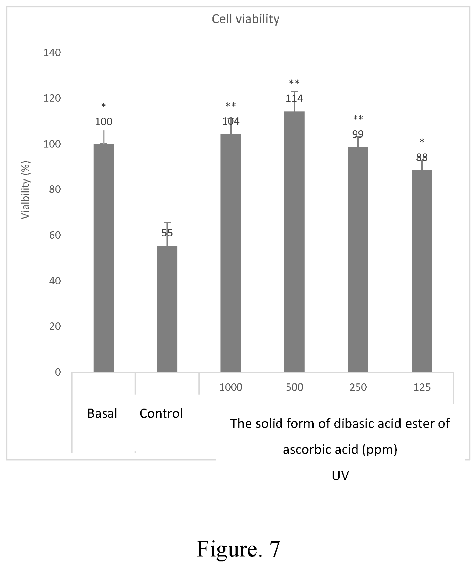

The results of repeated experiments are summarized in Figure 7. Under UV irradiation, the cell viability of the control group decreased from 100% to 55±10%. Compared to the control group, the solid form of dibasic acid ester of ascorbic acid of the present invention at concentrations of 125, 250, 500, and 1000 ppm restored cell viability to 88±4%, 99±5%, 114±9%, and 104±7%, respectively, and all showed statistically significant differences compared to the control group. The results show that the solid form of dibasic acid ester of ascorbic acid of the present invention has the effect of preventing and repairing UV-induced cell damage, with an effective concentration of 125~1000 ppm.

### Example 6: Efficacy Experiment Two (Intracellular Reactive Oxygen Species Content)

Cells were seeded into 48-well plates and cultured at 37°C and 5% CO2 for 24 hours. The cell line used was HEKn (Gibco, Cat#C-001-5C); the cell culture medium was Epi-Life (HKGS, Gibco, Cat#S0015 & Cat#MEPI500CA) containing HKGS (Human Keratinocyte Growth Supplement). The cell culture medium was replaced, with the experimental group receiving cell culture medium containing the solid form of dibasic acid ester of ascorbic acid of this invention, and the basal and control groups receiving cell culture medium without the solid form of dibasic acid ester of ascorbic acid of this invention. All cells were cultured at 37°C and 5% CO2 for 24 hours. Intracellular oxidative stress assay (DCFH-DA) was added: all groups were washed with sterile Dulbecco phosphate-buffered saline, and the intracellular oxidative stress assay (DCFH-DA) was added, followed by incubation for 1 hour. UV irradiation: Cells were washed with sterile Dulbecco phosphate-buffered saline, then irradiated with Dulbecco phosphate-buffered saline. Only the experimental group and the control group underwent UV irradiation; the basal group was cultured under normal conditions, with cells completely covered with aluminum foil to prevent UV irradiation. Post-UV irradiation treatment: The buffer solution was replaced with cell culture medium. The experimental group used cell culture medium containing the solid form of dibasic acid ester of ascorbic acid of this invention, while the basal and control groups used cell culture medium without the solid form of dibasic acid ester of ascorbic acid of this invention. Cells were cultured for another hour at 37°C and 5% CO2. Intracellular oxidative stress assay (ROS generation assay): The ROS content of each experimental group was measured using the intracellular oxidative stress assay (DCFH-DA), and the ROS content was calculated using Equation 2, with the control group data set as 100%. The comparison between groups was conducted using unpaired student t-tests. $\begin{matrix}ROS generation \left(%\right) \\ = \frac{Measured value of basal , control or experimental group}{Measured value of control group} \times 100 %\end{matrix}$ Equation 2

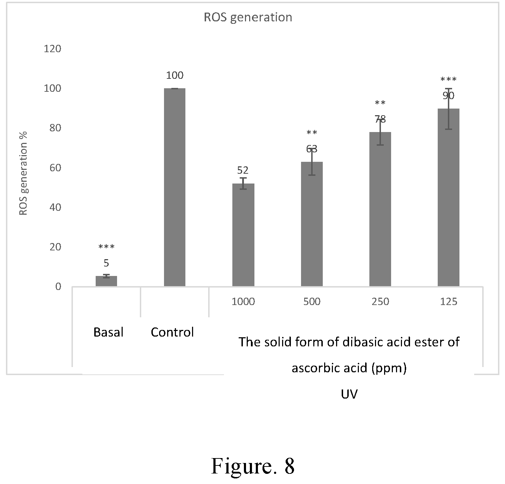

The results of repeated experiments using the above steps are summarized in Figure 8. The basal group consisted of untreated experimental materials and no UV irradiation, with ROS content representing the normal state. The control group consisted of untreated experimental materials irradiated with UV light, set at 100 %. Treatment with the solid form of dibasic acid ester of ascorbic acid of the present invention significantly reduced ROS content in a dose-dependent manner. At concentrations of 125 ppm, 250 ppm, 500 ppm, and 1000 ppm, ROS levels decreased from 100% in the control group to 90±10%, 78±6%, 63±7%, and 52±3 %, respectively. The results show that the solid form of dibasic acid ester of ascorbic acid of the present invention has a significant effect on reducing intracellular reactive oxygen species, with an effective concentration of 125~1000 ppm.

### Example 7: Efficacy Experiment 3 (Intracellular Interleukin - 6 Content)

Cells were seeded into 48-well plates and cultured at 37°C and 5% CO2 for 24 hours. The cell line used was HaCaT (AddexBio, Cat#T0020001); the cell culture medium was DMEM containing 10% fetal bovine serum. The cell culture medium was replaced with a buffer solution, and the cells were irradiated with UV light. The experimental group received a buffer solution containing the solid form of dibasic acid ester of ascorbic acid of this invention, while the basal and control groups received buffer solutions without the solid form of dibasic acid ester of ascorbic acid of this invention. Only the experimental group and the control group received UV irradiation; the basal group was cultured under normal conditions, with cells completely covered with aluminum foil to prevent UV irradiation. Post- UV irradiation treatment: The buffer solution was replaced with cell culture medium. The experimental group used cell culture medium containing the solid form of dibasic acid ester of ascorbic acid of this invention, while the basal and control groups used cell culture medium without the solid form of dibasic acid ester of ascorbic acid of this invention. Cells were cultured for 24 hours at 37°C and 5% CO2. Interleukin- 6 quantification: The level of interleukin-6 secreted by cells was tested using a human interleukin-6 Enzyme-linked immunosorbent assay (ELISA) kit. The relative interleukin-6 content was calculated using Equation 3, with the control group as 100%. Intergroup comparisons were performed using an unpaired Student's t-test. $\begin{matrix}Interleukin - 6 \left(%\right) \\ = \frac{Measured value of basal , control or experimental group}{Measured value of control group} \times 100 %\end{matrix}$ Equation 3

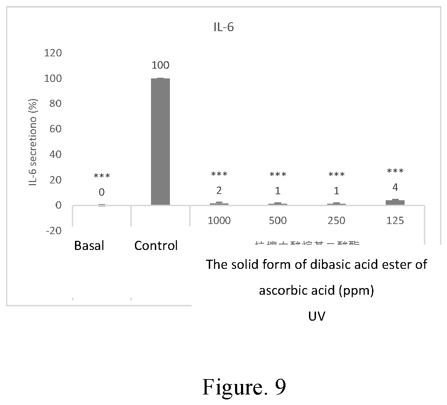

The results of repeated experiments using the above steps are summarized in Figure 9. The basal group consisted of untreated experimental materials and no UV irradiation, with IL-6 levels representing the normal state. The control group consisted of untreated experimental materials irradiated with UV light, set at 100 %. Treatment with the solid form of dibasic acid ester of ascorbic acid of this invention significantly reduced IL-6 levels in a dose-dependent manner. At concentrations of 125 ppm, 250 ppm, 500 ppm, and 1000 ppm, IL-6 levels decreased from 100% in the positive control group to 4 ± 1 %, 1 ± 1 %, 1 ± 1 %, and 2 + 1%, respectively. The results show that the solid form of dibasic acid ester of ascorbic acid of this invention has a significant effect in reducing intracellular inflammatory mediators, with an effective concentration of 125-1000 ppm.

### Example 8: Efficacy Experiment 4 (Collagen Content)

Cells were seeded into 48-well plates and cultured at 37°C and 5% CO2 for 24 hours. The cell line used was NHDF (PromoCell, Cat#C12302); the cell culture medium was FGM2 (PromoCell, Cat#C23230). Cell treatment was performed by replacing the cell culture medium. The experimental group received cell culture medium containing the solid form of dibasic acid ester of ascorbic acid of this invention, while the control group received cell culture medium without the solid form of dibasic acid ester of ascorbic acid of this invention. Cells were cultured at 37°C and 5% CO2 for 24 hours. Real-time quantitative polymerase chain reaction (RT-qPCR): The expression levels of collagen type I and type IV were tested using RT-qPCR. Relative collagen expression levels were calculated using Equation 4, with the control group as 100%. Intergroup comparisons were performed using an unpaired Student's t-test. $Collagen \left(%\right) = \frac{Measured value of control or expiremental group}{Measured value of control group} \times 100 %$ Equation 4

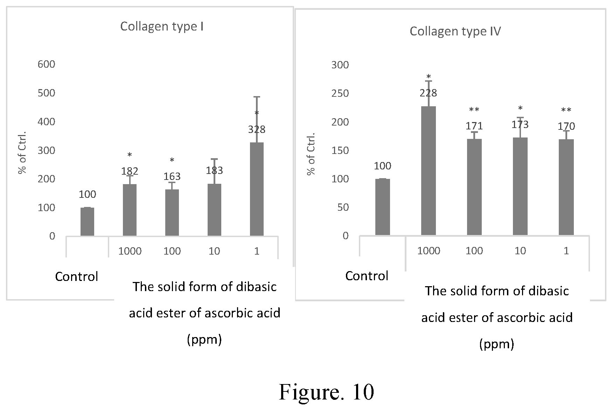

The results obtained from repeated experiments are summarized in Figure 10. The control group, which did not treat the experimental materials, represents the normal collagen content and is set at 100%. Treatment with the solid form of dibasic acid ester of ascorbic acid of this invention significantly increases collagen content. At 1 ppm, 10 ppm, 100 ppm, and 1000 ppm, the content of type I collagen increased from 100% to 328±159%, 183±87%, 163% 25%, and 182±30%, respectively; and the content of type IV collagen increased from 100% to 170± 15 % , 173 ± 36 %, 171 ± 12 %, and 228 ± 44%. The results showed that the solid form of dibasic acid ester of ascorbic acid of the present invention has a significant ability to increase the expression of collagen in fibroblasts, and the effective concentration is 1 ~1000 ppm.

### Example 9: Evaluation test of bases with different water activity values

The stability of various skin care compositions containing the solid form of dibasic acid ester of ascorbic acid has a XPRD pattern as shown in figure.1 in bases having different water activity values were evaluated. Specifically, the weight percentage of the solid form of dibasic acid ester of ascorbic acid has a XPRD pattern as shown in figure.1 in the representative base ranges from 0.1% to 20 wt.%; in this example, the weight percentage of the solid form of dibasic acid ester of ascorbic acid has a XPRD pattern as shown in figure.1 in the representative base is 1 wt.%; the water activity value of each base is between 0.05 and 0.79, as shown in Table 2.

**Table 2.**

| Water-activity | Base | Temperature | Degradation % of the solid form of the dibasic acid ester of ascorbic acid | |
|---|---|---|---|---|
| | | | 1 month | 4 months |
| 0.05 | Glycerin | Room temperature | 4.4% | 14.0% |
| 0.79 | Water: Glycerin (weight ratio 1:1) | Room temperature | 31.0% | 72.7% |
| 0.53 | Buffer (pH 4)* : Glycerol (weight ratio 1:9) | Room temperature | 9.3% | 16.9% |
| 0.61 | Buffer (pH 6 )** : Glycerol (weight ratio 1:9) | Room temperature | 6.7% | 16.3% |

| | | | | |
|---|---|---|---|---|
| * Buffer (pH 4 ): Sodium citrate buffer; **Buffer (pH 6 ): Phosphate buffer | | | | |

According to Table 2, when the water activity value of the representative base is 0.79, the degradation degree of the solid form of dibasic acid ester of ascorbic acid of the present invention is high. After being placed at room temperature for one month, HPLC analysis showed that its degradation percent reached 31%. In contrast, when the water activity value of the representative base is controlled to be less than 0.7, the stability of the solid form of dibasic acid ester of ascorbic acid of the present invention in the representative base can be improved. After being placed at room temperature for four months, its degradation percent is less than 20%, indicating that content of the solid form of dibasic acid ester of ascorbic acid is still more than 80%.

### Example 10: Gel

The composition of this example is shown in Table 3. The operation steps are as follows: First, mix the ingredients in phase A. After the solid form of dibasic acid ester of ascorbic acid is completely mixed, add phase A to phase B and mix evenly. The water activity value of the resulting gel is 0.75.

**Table 3**

| Phase | Ingredient(s) | weight ratio (%) |
|---|---|---|
| A | water | To 100.00 |
| | The solid form of dibasic acid ester of ascorbic acid | 1.00 |
| | Octyl glycol, phenoxyethanol, and hexanediol (Jeecide^{®} CAP-2) | 0.75 |
| B | Glycerin and glycerol acrylate/acrylic acid copolymers and 1,2-hexanediol (Carbojel CG-SF) | 85.00 |

### Example 11: Oil-in-water formulation ( O/W)

The composition of this example is shown in Table 4. The operation steps are as follows: First, xanthan gum was uniformly dispersed in water. After dispersion, phase C was added, followed by heating phase A, C, and B respectively. The oil phase was added to the aqueous phase after heating to 75°C. The resulting mixture was emulsified using a homogenizer and then cooled to 40°C. phase E was then added sequentially with stirring. The final cream product had a water activity value of 0.91. The cream product of this experimental example discolored after being left at room temperature for one month due to the degradation of the solid form of dibasic acid ester of ascorbic acid.

**Table 4.**

| Phase | Ingredient(s) | weight ratio (%) |
|---|---|---|
| A | water | To 100.00 |
| | Xanthan Gum | 0.10 |
| B | Cetyl alcohol / glyceryl stearic acid /PEG-75 stearate / cetyl alcohol polyether -20/ stearyl alcohol polyether -20 (EMULIUM^{®} DELTA MB) | 6.00 |
| | Cetearyl alcohol | 2.00 |
| | Polydimethylsiloxane | 1.00 |
| | Lauryl paraben | 3.00 |
| | Octyl dodecyl myristate | 5.00 |
| | Octyl dodecyl stearyl oxy stearate | 5.00 |
| C | Disodium EDTA | 0.05 |
| | Butylene glycol | 3.00 |
| D | Sodium hydroxide | 0.006 |
| E | The solid form of dibasic acid ester of ascorbic acid | 1.00 |
| | ethanol | 3.00 |
| | Phenoxyethanol | 0.85 |

### Example 12: Silicone Oil-in-Water Formulation (W/Si)

The composition of this example is shown in Table 5. The operation steps are as follows: Mix phase A and B thoroughly, then add the aqueous phase to the oil phase. The resulting mixture is emulsified using a homogenizing emulsifier, and its water activity value is less than 0.6.

**Table 5**

| Phase | Ingredient(s) | weight ratio (%) |
|---|---|---|
| A | water | To 100.00 |
| | Sodium chloride | 0.50 |
| | glycerin | 48.60 |
| | The solid form of dibasic acid ester of ascorbic acid | 1.00 |
| | Caprylyl glycol and Phenoxyethanol and Hexanediol (Jeecide^{®} CAP-2) | 0.75 |
| B | Cetyl PEG/PPG-10/1 polydimethylsiloxane | 2.00 |
| | Polydimethylsiloxane / vinyltrimethylsiloxane crosspolymer | 3.00 |
| | Polydimethylsiloxane | 10.00 |

### Example 13: Water-in-oil (W/O) Formulation

The composition of this example is shown in Table 6. The operating procedure is as follows: Phase A and B were mixed thoroughly, then the aqueous phase was added to the oil phase, and the resulting mixture was emulsified using a homogenizing emulsifier. The final cream product had a water activity value of 0.65. The cream product of this experimental example showed no change in appearance or color after being stored at room temperature for one month.

**Table 6**

| Phase | Ingredient(s) | weight ratio (%) |
|---|---|---|
| | water | To 100.00 |
| A | Sodium chloride | 0.50 |
| | glycerin | 48.60 |
| | The solid form of dibasic acid ester of ascorbic acid | 1.00 |
| | Caprylyl glycol and Phenoxyethanol and Hexanediol (Jeecide^{®} CAP-2) | 0.75 |
| B | Cetyl PEG/PPG-10/1 polydimethylsiloxane | 2.00 |
| | Jojoba ( Simondia chinensis ) seed oil | 4.00 |
| | Hydrogenated polydecene | 10.00 |
| | Polydimethylsiloxane | 8.00 |

### Example 14: Hydrogel-free gel

The composition of this example is shown in Table 7. The operation steps are as follows: First, mix the ingredients in phase A. After the the solid form of dibasic acid ester of ascorbic acid is completely mixed, add phase B and C in sequence and stir evenly. Finally, an anhydrous gel with a water activity value of less than 0.6 is obtained.

**Table 7**

| Group | Element | weight ratio (%) |
|---|---|---|
| A | glycerin | To 100.00 |
| | The solid form of dibasic acid ester of ascorbic acid | 1.00 |
| | Polyethylene glycol -400 (PEG-400) | 37.00 |
| B | Hydroxyethyl acrylate / sodium acrylamide dimethyl taurate copolymer | 1.50 |
| C | Caprylyl glycol and Phenoxyethanol and Hexanediol (Jeecide^{®} CAP-2) | 0.75 |

Obviously, many modifications and variations are possible in the above teachings. It is therefore to be understood that within the scope of the appended claims the present invention can be practiced otherwise than as specifically described herein. Although specific embodiments have been illustrated and described herein, it is obvious to those skilled in the art that many modifications of the present invention may be made without departing from what is intended to be limited solely by the appended claims.

## Claims

1. A solid form of dibasic acid ester of ascorbic acid, having a X-ray powder diffraction (XRPD) pattern that comprises characteristic peaks expressed in 2-theta degree at 7.2±0.2°, 14.1±0.2°, 18.5±0.2°, 18.7±0.2°, 19.8±0.2°, 22.4±0.2°, 23.9±0.2° and 25.4±0.2°.

2. The solid form of dibasic acid ester of ascorbic acid of claim 1, having a FTIR spectrum that comprises characteristic peaks at 3416±2 ^{cm-1},3172±2 ^{cm-1},2928±2 ^{cm-1}, 2857±2 ^{cm-1}, 1749±2 ^{cm-1}, 1737±2 ^{cm-1}, 1681±2 ^{cm-1}, 1347±2 ^{cm-1}, 1305±2 ^{cm-1}, 1146±2 ^{cm-1} and ^{761±2 cm-1}.

3. The solid form of dibasic acid ester of ascorbic acid of claim 1, having a DSC curve with one endotherm peak between 150 and 160°C.

4. A skincare composition, comprising 0.0001~20wt.% of the solid form of dibasic acid ester of ascorbic acid recited in claim 1~3 and 80~99.9999wt.% of a base relative to total weight of the skincare composition, and water activity value (aw) of the base is equal to or less than 0.7.

5. The skincare composition of claim 4, wherein the base is a form selected from the group consisting of water in oil, water in silicon, anhydrous gel and gel.

6. The skincare composition of claim 4, wherein the base comprises following ingredients: plant oils, mineral oils, polyols, silanes or their combinations.

7. A method for managing skin health, comprising topically applying a formulation to a surface of a skin in need, wherein the formulation comprises 0.0001~20wt.% of the solid form of dibasic acid ester of ascorbic acid recited in claim 1~3 relative to total weight of the formulation.

8. The method for managing skin health of claim 7, wherein the formulation is selected from the group consisting of cream, lotion, gel, powder and paste.

9. The method for managing skin health of claim 7, wherein the formulation is used for preventing fibroblasts or keratinocytes from UV damage.

10. The method for managing skin health of claim 7, wherein the solid form of dibasic acid ester of ascorbic acid recited in claim 1~3 is used for stimulating fibroblasts or keratinocytes to produce collagens.
